# FASCICULE DE BREVET EUROPEEN

(11) **EP 2 312 991 B1**
(45) Date de publication et mention de la délivrance du brevet: **28.03.2012**
(21) Numéro de dépôt: 09740463.6
(22) Date de dépôt: 13.08.2009
(51) Int. Cl.: A61B 1/05, A61B 5/00

(54) **CAPSULE AUTONOME DE DETECTION DESTINEE A ETRE INTRODUITE DANS UN CORPS HUMAIN OU ANIMAL**
ABGESCHLOSSENE NACHWEISKAPSEL ZUM EINFÜHREN IN EINEN MENSCHLICHEN ODER TIERISCHEN KÖRPER
SELF-CONTAINED DETECTION CAPSULE FOR INSERTION IN A HUMAN OR ANIMAL BODY

(30) Priorité: 14.08.2008 FR 0855586; 01.12.2008 US 118681 P
(43) Date de publication de la demande: 27.04.2011
(73) Titulaire: Quidd, 76800 St Etienne du Rouvray (FR)
(72) Inventeur: MASSONNEAU, Marc, F-27570 Tillieres Sur Avre (FR)
(74) Mandataire: Tanty, François
(86) Numéro de dépôt international: PCT/FR2009/051589
(87) Numéro de publication internationale: WO 2010/018350

(56) Documents cités:
- WO-A-2006/077529
- DE-A1-102007 001 925
- US-A1- 2005 266 074

## Description

La présente invention a pour objet des dispositifs et un procédé faisant intervenir des réactions de luminescence sur l'animal ou l'homme.

La présente invention s'applique plus particulièrement à l'observation de lumière émise par chimiluminescence ou bioluminescence.

Par « bioluminescence », on désigne la production et l'émission de lumière (visible ou non) résultant d'une réaction chimique impliquant une enzyme intra- ou extra-cellulaire au cours de laquelle de l'énergie chimique est convertie en énergie lumineuse.

Par « chimiluminescence », on désigne la production et l'émission de lumière (visible ou non) résultant d'une réaction chimique au cours de laquelle de l'énergie chimique est convertie en lumière.

Par ailleurs, par « fluorescence », on désigne l'émission de lumière (visible ou non) résultant de l'excitation d'une espèce fluorescente.

L'article Infect Immun. (2006 Sep; 74(9):5391-6. "In vivo bioluminescence imaging of the murine pathogen Citrobacter rodentium", Wiles S, Pickard KM, Peng K, MacDonald TT, Frankel G.) décrit une expérience dans laquelle des souris sont gavées avec des bactéries bioluminescentes exprimant la luciférase. La lumière émise par ces bactéries dans le tractus gastrointestinal a été observée avec l'imageur optique IVIS 50 de la société Xenogen, depuis l'extérieur du corps des souris.

L'article « In vivo monitoring of pancreatic β- cells in a transgenic mouse model », (Smith ST, Zhang H, Clermont AO, Powers AC, Kaufman DB, Purchio AF, West DB. Molecular imaging (2006), 5 (2) : 65-75) décrit un modèle animal de souris transgénique exprimant le gène de la luciférase régulé par le promoteur de l'insuline et l'utilisation de l'imageur IVIS100 de la société Xenogen pour mesurer la lumière émise par le pancréas de la souris.

L'article « A transgenic mouse model with a luciferase reporter for studying in vivo transcriptional regulation of the human CYP3A4 gene », (Zhang W, Purchio AF, Chen K, Wu J, Lu L, Coffee R, Contag PR, West DB, Metab Dispos (2003), 31: 1054-1064) décrit un modèle animal de souris transgénique exprimant le gène de la luciférase régulé par le promoteur de CYP3A4 et l'utilisation de l'imageur optique IVIS de la société Xenogen pour mesurer la lumière émise par le foie.

L'article « Differentiation and enrichment of hepatocyte-like cells from human embryonic stem cells in vitro and in vivo », (Duan Y, Catana A, Meng Yn Yamamoto N, he S, Gupta S, Gambhir SS, Zern MA, Stem cells (2007), 25 (12) : 3058-6) décrit un modèle animal obtenu par injection, dans un foie de souris, de cellules souches humaines embryonnaires infectées par un virus exprimant la luciférase et l'utilisation d'un imageur optique pour mesurer la lumière émise par le foie de la souris.

L'article « Dissecting tumor maintenance requirements using bioluminescence imaging of cell proliferation in a mouse glioma model », (Nature Medicine (2004), 10 : 1257-1260) décrit un modèle animal de souris transgénique possédant des tumeurs cervicales biolumineuses et l'utilisation d'un imageur optique pour mesurer la lumière émise par les tumeurs cervicales de la souris.

L'article « The characterization and hormonal regulation of kidney androgen-regulated protein (KAP)-luciferase transgenic mice », (Toxicological Sciences (2004), 46 : 266-277) décrit un modèle animal de souris transgénique exprimant le gène de la luciférase régulé par le promoteur de Kap et l'utilisation de l'imageur optique IVIS 100 de la société Xenogen pour mesurer la lumière émise dans le rein des souris.

L'article « Noninvasive bioluminescence imaging of normal and spontaneously transformed prostate tissue in mice », (Lyons SK, Lim E, Clermont AO, Dusich J, Zhu L, Campbell KD, Coffee RJ, Grasse DS, Hunter J, purchio T, Jenkins D, Cancer Res (2006) 66(9) : 4701) décrit un modèle animal de souris transgénique exprimant le gène de la luciférase régulé par le promoteur humain du gène de la prostate-specific antigen et l'utilisation de l'imageur optique IVIS 100-200 de la société Xenogen pour mesurer la lumière émise dans la prostate des souris.

L'utilisation d'imageurs optiques pour observer depuis l'extérieur du corps de l'animal la lumière émise intérieurement n'est pas entièrement satisfaisante, car la lumière est absorbée assez rapidement par les tissus, ce qui restreint l'étude à de petits mammifères.

De plus, les tissus diffusent fortement la lumière, ce qui rend difficile la localisation précise de la source.

Par ailleurs, des sondes fluorescentes Aposense et ICE-NIRF ont été utilisées pour visualiser l'apoptose, comme décrit dans « Molecular imaging of cell death in vivo by a small molecule probe », (Aloya R, Shirvan A, Grimberg H, reshef A, Levin G, Kidron D, Cohen A, Ziv I, Apoptosis (2006), 11 : 2089-2101) et « A novel method for imaging apoptosis using a caspase-1 near-infrared fluorescent probe », (Messerli SM, Prabhakar S, Tang Y, Shah K, Cortes ML, Murthy V, Weissleder R, Breakefield XO, Tung CH, Neoplasia (2004), 6 (2) : 95-105). Une autre sonde fluorescente Prosense 680 de la société Visen cible la cathepsine B exprimée dans de nombreux cancers.

Ces sondes fluorescentes peuvent générer par fluorescence une lumière qu'il est connu d'observer avec une capsule endoscopique.

Ainsi, l'abrégé disponible sur Internet avant publication « Gastrointest Endosc. 2008 May 20. Biochromoendoscopy: molecular imaging with capsule endoscopy for detection of adenomas of the GI tract » (Zhang H, Morgan D, Cecil G, Burkholder A, Ramocki N, Scull B, Lund PK) décrit la détection d'adénomes dans l'intestin de souris. La sonde fluorescente Prosense 680 de la société Visen est utilisée. Cette sonde, activable par la cathepsine B exprimée dans les adénomes, a permis de visualiser le cancer en utilisant une endoscopie par capsule. Les expériences ont été réalisées sur des intestins disséqués.

De nombreuses capsules absorbées oralement, et visant notamment à diagnostiquer des cancers des voies digestives d'un être humain sont par ailleurs connues.

La capsule proposée par la société MEDISCIENCE TECHNOLOGY® comporte sur sa face avant plusieurs diodes électroluminescentes (DEL) et un capteur CMOS. Cette capsule comporte également un microcontrôleur communiquant à distance avec un récepteur.

La capsule proposée par la société GIVEN IMAGING^{®} comporte une caméra et elle est agencée pour communiquer, lors de son transit par les voies digestives, avec un récepteur porté par le patient à la taille.

La capsule proposée par la société OLYMPUS^{®} comporte sur sa face avant six DELs blanches et un capteur CCD et elle est agencée pour transmettre à un moniteur distant des images de l'intérieur de l'intestin afin de détecter des anomalies éventuelles, telles que des saignements par exemple.

La société RF SYSTEM LAB^{®} propose, sous la dénomination SAYAKA^{™}, une capsule à double structure comportant une caméra et destinée à l'observation de l'intestin, la structure interne étant mise en rotation par un système d'aimants.

Le site http://www.popsci.com/how-it-works/article/2008-03/how-it-works-endoscope-camera-pill indique à propos de la capsule SAYAKA^{™} que celle-ci n'a pas besoin de moteur pour se déplacer dans l'intestin mais requiert cependant 50 milliwatts pour l'alimentation de la caméra, de l'éclairage et du processeur. Une pile étant trop volumineuse, la capsule tire sa puissance d'une charge par induction. Une veste portée par le patient contient une bobine et transmet en continu la puissance.

Sous la dénomination NORIKAT^{™}, cette même société RF SYSTEM LAB^{®} propose une capsule dépourvue de source d'énergie électrique et comportant quatre DELs et un capteur CCD pour acquérir des images de l'intérieur du corps humain. L'alimentation électrique des DELs et du capteur CCD se fait sans l'intermédiaire d'une connexion filaire.

La société SMARTPILL^{®} propose une capsule destinée à être avalée et comportant un capteur de pH, un capteur de pression et un capteur de température pour effectuer des mesures à l'intérieur des voies digestives.

La société INTELLIGENT MICROSYSTEM CENTER® propose une capsule comportant une ou plusieurs DELs et un système d'acquisition d'images comportant une lentille et un capteur d'images.

La publication « *A review of low-power wireless sensor microsystems for biomedical capsule diagnosis »* dont le contenu est incorporé par référence effectue une revue des systèmes de communication sans fils appliqués aux capsules biomédicales.

Il est également connu par la demande internationale WO 2004/032621 d'observer un conduit à l'aide d'une capsule introduite dans ce conduit pour détecter si des cellules cibles sont présentes. Cette capsule comporte une source de lumière éclairant le conduit et un capteur CMOS pour détecter la fluorescence émise par les cellules lorsqu'elles sont éclairées.

Avec les capsules mentionnées ci-dessus, on claire ou on excite, dans un intervalle de longueur d'ondes donné, la zone que l'on désire observer. La lumière émise par la source se superpose ainsi à la lumière émise en réponse par les composés organiques ciblés situés dans le champ de cet éclairage, ce qui réduit le rapport signal utile sur bruit et affecte ainsi la qualité de l'observation.

En outre, de telles capsules connues sont adaptées à l'observation de la surface d'un conduit humain mais non d'organes situés derrière de tels conduits.

On connaît par la demande WO 2004/045374 un procédé dans lequel on introduit dans le corps un marqueur radioactif, magnétique, fluorescent ou ultrasonique, émettant un signal détecté par une capsule avalée par le patient et comportant un détecteur adapté au marqueur utilisé.

WO 97/01986 divulgue un dispositif implantable permettant de mesurer, réguler, surveiller et transmettre des taux de constituants sanguins, comportant un capteur qui détecte in vivo le taux d'au moins un constituant sanguin, présent dans le tissu vasculaire d'un mammifère, et comprend une source de rayonnement allant de l'infrarouge jusqu'à la lumière visible. La source est conçue pour orienter ce rayonnement vers le tissu où il est modifié par son interaction avec celui-ci. La publication envisage de délivrer des doses de médicaments ou de moduler une fonction organique en réponse à un signal de commande découlant de la mesure du capteur.

Le brevet US 7 033 322 décrit un capteur implantable dans un vaisseau sanguin pour piloter une pompe à insuline.

Le document US-A-2005/0266074 concerne une capsule autonome comportant un détecteur optique agencé pour détecter une lumière et des moyens d'enregistrement des données relatives à la lumière détectée.

Il existe un besoin pour bénéficier de solutions performantes pour l'étude, le dépistage et le traitement de maladies, entre autres.

### Résumé

L'invention propose une nouvelle capsule à introduire dans le corps humain ou animal et des utilisations de cette capsule à de multiples fins, de diagnostic ou thérapeutiques.

La capsule peut être destinée à être utilisée pendant une courte période dans le corps, par exemple le temps du transit intestinal dans le cas où la capsule est avalée.

La capsule peut encore être laissée plus longtemps dans le corps, par exemple de façon à fournir périodiquement une information sur un état physiologique de la personne.

L'introduction de la capsule peut s'effectuer dans un conduit, par exemple le conduit digestif, ou dans un tissu ou organe.

Le terme « capsule » ne doit pas être interprété limitativement et couvre tout dispositif fixe ou mobile permettant de réaliser les fonctions recherchées après introduction dans le corps. Le terme « capsule » peut ainsi couvrir un implant laissé immobile dans le corps pendant une durée plus ou moins longue, voire qui n'est jamais enlevé.

### Capsule

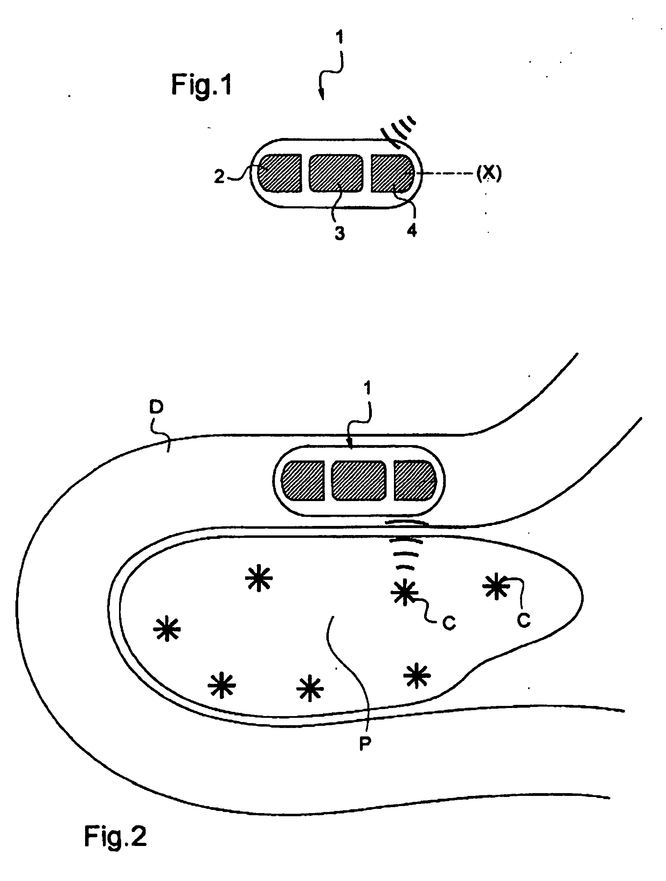

On a représenté très schématiquement à la figure 1 un exemple de capsule 1 réalisée conformément à l'invention.

La capsule peut présenter des formes diverses, selon notamment qu'elle est destinée à se déplacer ou non. Une forme généralement aplatie peut faciliter un positionnement statique, tandis qu'une forme de révolution peut faciliter son transit.

L'invention a en effet pour objet, selon un premier de ses aspects, une capsule autonome de détection destinée à être introduite dans le corps humain ou animal, notamment dans un conduit de celui-ci, et dépourvue de source lumineuse pour illuminer le champ d'observation, notamment pendant la détection, la capsule pouvant comporter, comme illustré à la figure 1 :
- un détecteur optique 4 ayant un champ d'observation et agencé pour détecter une lumière émise par bioluminescence ou chimiluminescence, sans excitation lumineuse du champ d'observation du détecteur optique par la capsule, et
- des moyens 3 d'enregistrement, de traitement et/ou de transmission de données relatives à la lumière détectée par le détecteur optique.

On désigne par « capsule autonome » une capsule qui, lorsqu'elle est introduite dans le corps humain ou animal, n'est reliée à l'extérieur du corps par aucune connexion filaire.

La capsule autonome est par exemple inerte, lorsqu'elle est introduite dans le corps humain ou animal. On désigne par « inerte » une capsule qui n'interagit pas avec le corps humain ou animal lorsqu'elle est introduite dans ce dernier.

La capsule peut comporter un moyen 2 d'alimentation en énergie.

Par « source lumineuse » on entend une source capable de produire un rayonnement significatif dans le visible, l'ultraviolet ou le proche infrarouge, utilisée pour la détection. Il s'agit par exemple d'une LED ou similaire.

L'absence d'excitation lumineuse permet d'éviter que l'observation du signal utile, correspondant à la lumière émise par bioluminescence ou chimiluminescence, par le détecteur optique ne soit perturbée par une excitation lumineuse préalable ou simultanée du champ d'observation du détecteur optique. Le rapport signal/bruit s'en trouve amélioré. L'amélioration du rapport signal sur bruit est susceptible de simplifier le traitement effectué pour extraire des données de détection de l'information exploitable et d'accroître la sensibilité.

En outre, l'absence de source lumineuse permet d'augmenter l'autonomie ou, à autonomie équivalente, de réduire la taille d'une source d'énergie électrique embarquée le cas échéant dans la capsule, ce qui peut permettre de réduire l'encombrement de la capsule et, par conséquent, faciliter son transit. Cela peut également permettre une alimentation par induction à une distance plus grande, car l'énergie demandée par la capsule peut être plus faible.

L'absence de source lumineuse contribue également à réduire l'encombrement de la capsule et peut permettre par exemple, à encombrement égal, d'utiliser un détecteur optique ayant une plus grande sensibilité ou un champ d'observation plus étendu. Cela peut permettre encore de disposer d'un plus grand nombre de capteurs sur la capsule, afin d'obtenir une meilleure sensibilité et/ou une meilleure localisation de l'origine de la lumière détectée, par exemple autour de son axe.

Par ailleurs, la détection de lumière émise par luminescence autogène est susceptible de faciliter l'interprétation des données issues de la détection. Il n'est plus nécessaire de faire visualiser par un spécialiste un enregistrement vidéo pris par une caméra de la capsule pour analyser les données de détection, comme c'est le cas de certaines capsules connues, puisque la capsule peut par exemple, dans le cas d'une application au diagnostic, détecter ou non une luminescence autogène. Seulement en cas de détection d'une luminescence autogène, des examens complémentaires pourront être effectués, ou la capsule pourra accomplir une ou plusieurs actions prédéfinies.

### Enveloppe de la capsule

En fonction de l'application visée, l'enveloppe de la capsule pourra être réalisée dans un ou plusieurs matériaux différents. Par exemple, dans le cas où la capsule est avalée, il suffit que l'enveloppe ne soit pas toxique et résiste au transit intestinal. Quand la capsule est destinée à être laissée à demeure dans le corps pendant plusieurs années, l'enveloppe doit présenter une biocompatibilité suffisante.

La surface extérieure de la capsule est par exemple définie par un ou plusieurs matériaux biocompatibles, comme une résine, par exemple de polyether-terketone.

La capsule peut comporter une enveloppe résistant à certains paramètres physiologiques du conduit digestif, notamment à un milieu acide.

La capsule peut comporter un revêtement de dioxyde de manganèse (MnO₂) afin de créer un effet laxatif permettant un transit dans le conduit digestif plus facile et plus rapide. Un tel exemple de revêtement de dioxyde de manganèse est divulgué dans la demande internationale WO 2004/045374.

L'enveloppe de la capsule ne doit pas gêner la réception de la lumière par le détecteur optique, dans le cas où le détecteur est situé derrière l'enveloppe. L'enveloppe de la capsule peut être réalisée dans un ou plusieurs matériaux transparents à la longueur d'onde d'émission de la luminescence autogène, par exemple transparents au proche infrarouge. L'enveloppe de la capsule est par exemple formée par une matière plastique ou une résine moulée sur les composants internes de la capsule, cette matière plastique ou résine étant à la fois suffisamment biocompatible et suffisamment transparente au proche infrarouge ou autre longueur d'onde d'émission de la luminescence infrarouge ou visible. Le cas échéant, la surface externe de l'enveloppe peut avoir une forme calculée pour concentrer la lumière sur un ou plusieurs capteurs du détecteur optique, afin par exemple d'augmenter le champ d'observation.

Une portion de la surface extérieure de la capsule peut être définie par une fenêtre du détecteur optique, par exemple une ou plusieurs lentilles du détecteur optique. Le reste de la surface extérieure peut être défini par exemple par une matière moulée au contact de cette ou ces lentilles, afin d'assurer leur maintien et l'étanchéité de la capsule.

La capsule doit permettre de récupérer des données issues de la détection. L'enveloppe de la capsule est réalisée de façon à ne pas gêner cette récupération des données qu'elle s'effectue avec contact ou sans contact.

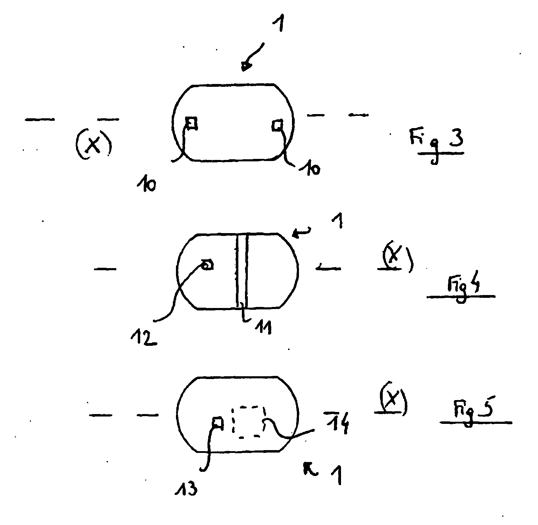

L'enveloppe de la capsule peut être totalement physiologiquement inerte vis-à-vis du milieu environnant. L'enveloppe de la capsule peut encore être configurée pour interagir avec son environnement, par exemple de façon programmée ou déclenchée, à des fins de diagnostic ou thérapeutiques.L'enveloppe de la capsule peut par exemple comporter, comme représenté à la figure 3, au moins deux électrodes 10 permettant d'effectuer, par exemple, des mesures de conductivité électrique ou d'émettre un champ électrique dans le milieu environnant. La capsule peut par exemple émettre une énergie radiofréquence dans un but curatif, en réponse à une détection représentative d'une activité cancéreuse, en vue de détruire lesdites cellules. L'énergie nécessaire à la production du champ radiofréquence peut éventuellement être obtenue par induction, en plaçant la capsule dans un champ électromagnétique adapté.

L'enveloppe de la capsule peut encore être réalisée de façon à transmettre de la chaleur au milieu environnant, dans un but curatif. La chaleur peut être produite au niveau de la surface extérieure de la capsule ou intérieurement à l'enveloppe de la capsule.La capsule peut comporter un moyen de production de chaleur pour élever une région au moins de la surface extérieure de la capsule à une température supérieure ou égale à 40 °C.

Dans un exemple de mise en oeuvre représenté à la figure 4, le moyen de production de chaleur comporte un anneau de court-circuit 11 qui, placé dans un champ magnétique fluctuant externe au corps, reçoit de l'énergie par induction et dégage par effet Joule de la chaleur. La capsule peut comporter un capteur de température 12 et transmettre une information représentative de la température de surface de la capsule au dispositif émetteur du champ externe afin de réguler le champ magnétique et maintenir la température à une valeur optimale pour le traitement à effectuer. L'anneau de court-circuit Il peut être une bague métallique externe à la capsule ou interne à celle-ci.

La capsule peut comporter un ou plusieurs actionneurs permettant de faire varier, par exemple augmenter ou diminuer ses dimensions, une fois actionné(s). Cela peut permettre, par exemple, d'utiliser la capsule pour réaliser une compression d'une zone prédéfinie en cas de détection de lumière représentative d'une activité cancéreuse. La dilatation de la capsule peut aider au confinement des cellules tumorales ou réduire leur irrigation sanguine en l'attente d'une intervention chirurgicale ou de radiothérapie, par exemple.

L'enveloppe de la capsule peut comporter un actif, par exemple un anticancéreux, encapsulé, libérable par exemple en élevant la température de la capsule. Dans ce cas, la capsule comporte par exemple l'anneau de court-circuit 11 précité, ce qui permet de chauffer la capsule et de provoquer la libération de l'actif au plus près de la cible à atteindre.

En variante, la capsule peut envoyer sur la cible à atteindre une énergie ultrasonore, radiofréquence, transportée par un faisceau de particules ionisantes, de neutrons, de rayons γ ou X.

### Moyen d'alimentation en énergie de la capsule

La capsule peut comporter une source d'énergie électrique embarquée. La capacité de la source d'énergie électrique est par exemple comprise entre 50mAh et 400mAh, étant choisie en fonction de la taille et du nombre de composants de la capsule à alimenter et de l'autonomie recherchée.

La source d'énergie électrique embarquée est par exemple une pile, par exemple une pile bouton, ou un accumulateur, par exemple de type lithium polymère.

L'alimentation électrique de la capsule peut encore s'effectuer par induction, la capsule comportant une bobine placée dans un champ magnétique variable généré à l'extérieur du corps. Par exemple, le patient ayant avalé la capsule peut porter à la taille un dispositif créant un champ électromagnétique permettant de fournir de l'énergie à la capsule.

L'alimentation de la capsule peut encore s'effectuer par un système d'alimentation hybride accumulateur/induction, en réalisant la capsule avec un accumulateur et avec une bobine destinée à être soumise à un champ électromagnétique afin de générer un courant électrique permettant de recharger l'accumulateur. L'absence de source lumineuse dans la capsule peut faciliter l'implantation dans la capsule d'un tel système d'alimentation hybride, pour un encombrement donné.

Lorsque la capsule comporte une bobine permettant de générer un courant électrique utile pour alimenter des composants de la capsule, le couplage inductif entre cette bobine et le système générant le champ électromagnétique peut être modifié par la capsule, par exemple en court-circuitant la bobine, en vue de transmettre des données à l'extérieur du corps dans lequel est placée la capsule.

L'alimentation de la capsule peut encore, le cas échéant, s'effectuer par une transformation de mouvement en électricité ou par une transformation de la chaleur du corps en énergie électrique.

### Détecteur optique

Le détecteur optique est par exemple sensible à une lumière visible (380-780 nm) ou non visible.

Le détecteur optique est par exemple sensible au proche infrarouge. On désigne par « proche infrarouge » les radiations de longueurs d'onde comprises entre 600 et 900 nm, notamment 650 et 850 nm.

Le détecteur peut-être agencé pour n'observer que selon une direction d'observation ou en variante selon plusieurs directions, auquel cas le détecteur optique est dit multiaxe.

Le détecteur optique peut être formé d'un ou d'une multitude de capteurs, par exemple une ou plusieurs photodiodes ou phototransistors.Le détecteur optique peut comporter une pluralité de capteurs optiques agencés pour localiser l'émission de lumière relativement à la capsule.

Le détecteur optique peut comporter des capteurs disposés de différentes façons, au coeur de la capsule ou à sa surface.

Les capteurs utilisés sont par exemple disposés selon une surface de détection convexe ou hémisphérique.

En variante, les capteurs sont disposés selon une surface de détection plane.

Toute la surface de la capsule est par exemple recouverte de capteurs, notamment de micro-capteurs.

Un système de lentilles et/ou de réflecteurs approprié peut permettre une observation multiaxe tout en ayant une surface de détection plane, afin d'obtenir un champ d'observation par exemple supérieur à 180°. Le détecteur optique est par exemple agencé de manière à pouvoir atteindre une sensibilité allant jusqu'à la détection d'un seul photon, grâce à la présence d'une structure amplificatrice de la lumière au sein du détecteur optique. Le détecteur peut comporter au moins un capteur à amplification optique, par exemple une ou plusieurs photocathodes afin de libérer des électrons sous l'effet de la lumière incidente, électrons qui pourront être amplifiés par un phénomène d'avalanche.

Le détecteur comporte par exemple un ou plusieurs capteurs du type EMCCD, ICCD, EBCCD ou CMOS.

La détection optique peut comporter, le cas échéant, un ou plusieurs filtres sélectifs, de bande passante choisie en fonction de la longueur d'onde de la lumière produite par luminescence autogène.

Le ou les filtres sélectifs peuvent être obtenus, le cas échéant, par un dépôt multicouche formé sur l'enveloppe de la capsule.

Le détecteur optique peut comporter une matrice de capteurs ou une ou plusieurs barrettes de capteurs ou un anneau de capteurs.

### Moyens d'enregistrement, de traitement et/ou de transmission des données

Les données issues du détecteur peuvent être sous forme de signal analogique ou numérique, selon que le détecteur comporte un ou plusieurs circuits intégrés de mise en oeuvre des signaux et de lecture des capteurs.

La capsule peut comporter, le cas échéant, un convertisseur analogique/numérique.

Les données de détection peuvent, le cas échéant, subir un traitement analogique, par exemple d'intégration. Par exemple, le détecteur comporte une photodiode et le courant issu de la photodiode, proportionnel à la quantité de lumière reçue, peut charger un condensateur qui réalise ainsi l'intégration du signal.

La capsule peut comporter une unité de traitement, par exemple un microcontrôleur ou microprocesseur, pour traiter et mémoriser les données relatives à la lumière détectée par le détecteur optique.

La capsule peut comporter un émetteur, par exemple radiofréquence, pour communiquer les données traitées par le détecteur ou l'unité de traitement intégrée à la capsule à un récepteur externe situé à l'extérieur du corps humain ou animal dans lequel la détection a lieu.

En variante, la capsule est dépourvue d'unité de traitement et les données relatives à la lumière détectée par le détecteur optique sont directement transmises en temps réel à un récepteur externe dans lequel le traitement des données a lieu.La capsule peut comporter une mémoire électronique pour enregistrer des données de détection. Lesdites données de détection peuvent être transférées à distance, sur requête externe. La capsule peut comporter un moyen pour déclencher automatiquement des phases de détection périodiquement.

Le traitement opéré par la capsule peut consister en une amplification et/ou mise en forme du signal délivré par le détecteur, en vue de son envoi sous forme de signal analogique ou numérique au récepteur externe.

Le traitement peut être plus complexe, et comporter par exemple l'intégration du signal délivré par le détecteur et/ou l'application de filtres et/ou transformées mathématiques diverses.

Que le traitement soit effectué dans la capsule et/ou le récepteur externe et/ou dans un ordinateur auquel le récepteur externe transmet les données relatives à la détection effectuée par le détecteur, le traitement peut comporter une intégration du signal dans le temps, afin de connaître la quantité globale de signaux reçus par le détecteur.

La capsule peut recevoir des informations lors de son activation qui pourront servir au traitement effectué au sein de la capsule.

Le traitement peut tenir compte d'informations transmises par des capteurs annexes n'appartenant pas au détecteur optique, par exemple des capteurs de mouvement ou d'orientation.

La capsule peut, comme représenté à la figure 5, commander au moins un actuateur embarqué 13. Le traitement des données effectué par la capsule peut viser à déclencher automatiquement ou sur commande l'actuateur 13 lorsque des conditions prédéfinies sont remplies. L'actuateur 13 vise par exemple à ouvrir un réservoir 14 faisant diffuser une substance à l'extérieur de la capsule ou à dilater la capsule.

### Transmission des données

La capsule peut comporter un émetteur radiofréquence. Ce dernier peut servir à transmettre en temps réel des données de détection ou en variante peut n'être utilisé qu'après récupération de la capsule, pour télécharger le contenu d'une mémoire dans laquelle ont été stockées les données de détection.

La transmission de données depuis la capsule peut s'effectuer par paquets, ce mode de transmission étant plus économe sur le plan énergétique.

L'émetteur radiofréquence émet par exemple dans la bande de fréquence 20MHz-40MHz, mais d'autres fréquences sont utilisables.

Lorsque la capsule est récupérée après avoir effectué une phase de détection dans le corps, l'information relative à la détection peut être transmise à un lecteur autrement que par une transmission radiofréquence, par exemple par transmission infrarouge ou par un signal électrique véhiculé par une ligne électrique, après établissement d'une liaison électrique entre la capsule et le lecteur.

La capsule peut comporter un récepteur de données.

Ces données reçues sont par exemple relatives à des paramètres de détection, par exemple le traitement à effectuer sur les données issues du détecteur optique. Le récepteur peut aussi recevoir des données commandant la mise en marche du détecteur optique, par exemple afin de ne mesurer la lumière émise que pendant le trajet de la capsule devant un organe donné.

La capsule, lorsqu'elle est implantée durablement dans le corps, peut encore recevoir des données concernant la fréquence avec laquelle la détection doit avoir lieu.

Lorsque la capsule est alimentée par induction, le champ électromagnétique d'induction peut être modulé pour transmettre des données à la capsule.

### Autres capteurs

La capsule peut comporter un ou plusieurs capteurs non optiques.

La capsule peut comporter des capteurs pour mesurer des paramètres physiologiques, par exemple relatifs au conduit dans lequel elle est introduite, par exemple un capteur de pH, de conductivité, de température ou de pression. La capsule peut comporter des biocapteurs, par exemple de type puce à ADN.

La capsule peut comporter un capteur intégrant une source lumineuse qui reste éteinte lors d'une détection de luminescence autogène.

### Substances libérées par la capsule

On peut profiter de la présence de la capsule dans le corps pour libérer une ou plusieurs substances. Cette ou ces substances peuvent être libérées dans un but thérapeutique ou de diagnostic. La libération peut être programmée dans le temps, être automatique en réponse à une détection de luminescence et/ou être commandée depuis l'extérieur du corps, par exemple par un signal radiofréquence.

La capsule peut comporter un compartiment contenant une substance destinée à être libérée lorsque la capsule est dans le corps humain ou animal. Ce compartiment peut par exemple communiquer par l'intermédiaire d'une micro vanne avec l'extérieur de la capsule.

La capsule peut par exemple libérer une substance utile pour la détection et destinée à se fixer à des cellules cibles de façon à provoquer l'émission par bioluminescence par lesdites cellules cibles d'un rayonnement lumineux détectable par la capsule. Une telle substance met par exemple en oeuvre le couple renilla luciférase/coelenterazine.

Dans l'exemple où le rayonnement lumineux détectable par la capsule est provoqué par une réaction de chimiluminescence, la substance utile pour la détection est par exemple choisie selon l'enseignement de la demande internationale WO 2006/129036 au nom de la Demanderesse.

La substance libérée par la capsule peut encore avoir des vertus curatives. Il peut s'agir par exemple d'un anti-cancéreux.

La capsule peut être agencée pour libérer automatiquement une substance contenue dans la capsule en cas de détection de lumière. En variante, la capsule peut libérer une substance sur ordre reçu de l'extérieur. Une image du corps dans lequel se trouve la capsule est par exemple acquise par imagerie médicale et, lorsque la capsule se trouve dans une région donnée du conduit, l'ordre de libération de la substance est transmis à la capsule.

La capsule peut être agencée pour libérer la substance selon des conditions prédéfinies détectées par des capteurs non optiques.

La capsule comporte par exemple un capteur sensible au pH et la libération de la substance s'effectue lorsque la valeur de pH mesurée atteint un seuil prédéfini, représentatif de la présence de la capsule dans une région donnée.

Dans une autre variante, la capsule comporte un capteur détectant la présence d'une enzyme spécifique et la substance est libérée lorsque ladite enzyme est présente dans des quantités supérieures ou inférieures à un seuil prédéfini.

### Utilisation de la capsule comme assistance au traitement

La capsule peut être utile pour améliorer la précision d'un traitement opéré depuis l'extérieur du corps portant la capsule. Par exemple, la capsule peut signaler une détection de lumière autogène, par exemple représentative d'une activité métastatique, alors qu'elle est à un emplacement donné de son trajet dans le corps. La connaissance de sa position au moment du signalement peut servir à focaliser un faisceau d'ultrasons, de radiofréquences, une onde de choc ou des rayons X, γ, de neutrons, protons ou hadrons sur une cible dont les coordonnées sont connues précisément grâce à la capsule.

La capsule peut contenir, dans un exemple de mise en oeuvre de l'invention, un matériau permettant la formation *in situ* d'un isotope radioactif au niveau de la capsule, sous l'effet d'un bombardement de particules énergétiques depuis l'extérieur du corps.

### Procédé de détection

L'invention a encore pour objet, selon un autre de ses aspects, un procédé d'observation du corps humain ou animal, comportant les étapes définies par la revendication 15.

Le procédé peut comporter l'étape consistant à sacrifier l'animal, par exemple après l'observation. Une partie de la paroi du conduit ou de l'organe situé derrière le conduit ayant émis la lumière provenant d'une réaction de bioluminescence ou de chimiluminescence peut ensuite être prélevée, le cas échéant. La capsule peut être récupérée avant ou après avoir sacrifié l'animal.

### Traitement de la prostate

On peut laisser à demeure une capsule pour détecter périodiquement ou sur commande une éventuelle émission lumineuse liée à un marqueur précédemment absorbé.

Il s'agit par exemple du marqueur fluorescent PMSA tel que décrit dans la publication Humblet et al., 2005 Molecular Imaging.

### Ensemble à double capsules

L'invention a encore pour objet, selon un autre de ses aspects, un ensemble de capsules autonomes, comportant :
- une première capsule telle que définie ci-dessus,
- une deuxième capsule distincte de la première et agencée pour libérer une substance destinée à provoquer l'émission par chimiluminescence ou bioluminescence de lumière en présence d'une cible prédéfinie, cette lumière étant détectable par la première capsule.

La deuxième capsule est par exemple avalée avant la première et libère à un emplacement prédéfini une substance qui va générer une lumière détectable par la première capsule. La substance libérée par la deuxième capsule peut suivre un passage hépatique ou non.

Un ensemble de capsules autonome est aussi décrit, par exemple contenues initialement dans un même conditionnement, comportant :
- une première capsule agencée pour détecter une lumière émise par fluorescence, et comportant à cet effet une source lumineuse pour exciter la fluorescence et,
- une deuxième capsule agencée pour libérer un marqueur fluorescent qui se fixe sur des cellules cibles.

Le marqueur fluorescent est par exemple l'un de ceux de l'état de la technique commenté précédemment.

La deuxième capsule peut comporter un compartiment contenant la substance à libérer et le compartiment peut s'ouvrir en réponse à un ordre reçu de l'extérieur du corps ou l'ouverture peut résulter de la détection de certains paramètres d'environnement de la capsule, grâce à au moins un capteur présent sur la deuxième capsule, par exemple un capteur de pH.

On peut éventuellement utiliser plusieurs marqueurs émettant à des longueurs d'ondes différentes, pour effectuer une détection sélective.

### Procédé d'observation à l'aide d'un cathéter

Un procédé d'observation du corps humain ou animal est aussi décrit, comportant l'étape consistant à :
- observer une lumière provenant d'une réaction de bioluminescence ou de chimiluminescence à l'aide d'un cathéter introduit dans un conduit dudit corps.

Le procédé peut comporter l'étape préalable consistant à provoquer une réaction de bioluminescence ou de chimiluminescence dans ledit corps.

Le procédé peut être appliqué à un animal et être suivi du sacrifice de l'animal.

Le cathéter comporte par exemple un tube à l'extrémité duquel est monté un détecteur optique tel que décrit précédemment. Le diamètre du tube est adapté à son insertion dans la zone à explorer.

Le cathéter peut encore comporter une ou plusieurs fibres optiques pour transmettre la lumière émise par le corps à un détecteur optique placé à l'extérieur du corps.

### Procédés de dépistage d'une pathologie

Un procédé de dépistage d'une pathologie dans un corps humain ou animal est aussi décrit, comportant les étapes consistant à :
- détecter une lumière provenant d'une réaction de bioluminescence ou de chimiluminescence à l'aide d'au moins une capsule telle que définie ci-dessus ou d'au moins un cathéter comportant un détecteur optique et, introduit(e) dans le corps,
- analyser la lumière détectée et,
- évaluer si le corps humain ou animal est atteint de la pathologie.

La pathologie est par exemple le diabète ou un cancer de l'un des organes du conduit digestif ou d'un organe situé à proximité du conduit digestif

Deux réactions de bioluminescence ou de chimiluminescence émettant à des longueurs d'onde différentes peuvent être provoquées à l'aide de deux substances distinctes, préalablement introduites dans le corps, par exemple deux 1,2 dioxetanes portant des substituants différents permettant de régler les longueurs d'onde d'émission ou plusieurs couples luciférine/luciférase.

La détection par la capsule ou par le cathéter des rayonnements lumineux correspondant aux différentes longueurs d'onde peut permettre de déceler la pathologie avec un risque d'erreur moindre.

Un procédé d'observation d'une réaction de bioluminescence ou de chimiluminescence dans un corps humain ou animal est aussi décrit dans lequel
- on introduit dans un conduit dudit corps, par exemple le conduit digestif, une capsule autonome de détection telle que définie ci-dessus, comportant :
   - un détecteur optique agencé pour détecter la lumière émise par bioluminescence ou chimiluminescence par ledit corps, et
   - des moyens d'enregistrement, de traitement et/ou de transmission des données relatives à la lumière détectée par le détecteur optique,
- on reçoit à l'aide d'un récepteur externe au corps des données relatives à la lumière détectée par le détecteur optique de la capsule.

Dans ce procédé, on peut acquérir par imagerie médicale une image du corps dans lequel se trouve la capsule.

La capsule peut notamment émettre en temps réel un signal indiquant que le détecteur optique reçoit la lumière qu'il est censé détecter, et l'on peut procéder à ce moment-là à une imagerie du corps, par exemple par radiographie, tomographie par rayons X, IRM ou échographie, afin de localiser la capsule et connaître plus précisément la zone d'émission de la lumière.

Il s'agit par exemple d'une émission de lumière autogène ou par fluorescence. En cas de détection représentative d'une présence tumorale ou potentiellement tumorale, la capsule peut exercer une action thérapeutique ou préventive, soit directement, par exemple en libérant un actif, soit indirectement, par exemple en chauffant sous l'effet d'un champ électromagnétique externe.

### Exemple d'observation d'un organe à travers la paroi d'un conduit

On a représenté à la figure 2 un exemple d'application de l'invention à l'observation du pancréas.

Dans cet exemple, la capsule 1 est introduite dans l'intestin et passe dans le duodénum D. Comme on peut le voir sur la figure 2, une partie importante du pancréas P est bordée par le duodénum chez l'humain mais également chez de nombreux animaux.

Le détecteur optique 4 détecte dans cet exemple la lumière émise par chimiluminescence ou bioluminescence par des cellules C du pancréas. La quantité de lumière reçue, déterminable par l'aire sous la courbe du signal détecté par la capsule, est par exemple proportionnelle à la masse de cellules béta du pancréas, et peut fournir une information utile sur le diabète.

### Implantation de la capsule après une intervention chirurgicale

Une capsule selon l'invention peut être implantée dans le corps humain ou animal afin d'effectuer une surveillance et détecter par exemple l'efficacité de certaines substances dans un traitement ou détecter l'apparition de cellules cancéreuses.

La capsule peut être implantée, dans l'exemple du cancer du sein, dans un ganglion sentinelle, et peut être activée périodiquement pour fournir une information utile.

La capsule est par exemple telle que définie ci-dessus, agencée pour détecter une lumière émise par luminescence autogène. En variante, selon cet aspect de l'invention, la lumière peut encore être émise par fluorescence exogène, et la capsule peut alors comporter une source lumineuse capable d'exciter cette fluorescence.

La capsule peut être alimentée électriquement par induction lorsqu'il est nécessaire de détecter la lumière et être laissée en sommeil à l'état inactif hors des périodes de détection.

La capsule peut être implantée sur des personnes à risque, passé un certain âge.

### Détection post-thérapeutique

Une capsule selon l'invention peut être utile pour effectuer une détection post-thérapeutique, et vérifier par exemple l'absence de cellules cancéreuses en fin de traitement. La capsule peut être laissée en place à la fin du traitement, et être activée périodiquement pour vérifier l'absence de retour de la maladie.

### Variantes

Une capsule autonome de détection est aussi décrit comportant :
- une enveloppe permettant son introduction dans le corps humain ou animal,
- un détecteur optique à structure amplificatrice de la lumière,
- des moyens d'enregistrement, de traitement et/ou de transmission de données relatives à la lumière détectée par le détecteur optique.

Le détecteur optique peut être sensible au proche infrarouge.

Un procédé de détection de luminescence au sein d'un corps humain ou animal est aussi décrit, dans lequel on observe à l'aide d'un détecteur de la capsule ci-dessus, une bioluminescence ou chimiluminescence, sans excitation optique du champ d'observation. Le procédé peut être appliqué à l'animal. Le cas échéant, l'observation de la bioluminescence ou de la chimiluminescence peut être précédée ou suivie d'un sacrifice de l'animal.

L'observation peut s'effectuer dans le but d'apprécier l'efficacité thérapeutique d'un traitement, notamment oncologique.

La capsule peut être utilisée pour effectuer un traitement thérapeutique.

Une technique d'imagerie peut être mise en oeuvre pour acquérir au moins une image du corps au moment où une luminescence est détectée.

Le procédé peut comporter l'étape selon laquelle une énergie est envoyée sur une région du corps d'où provient la luminescence détectée par la capsule afin de provoquer la destruction des cellules à l'origine de la luminescence, notamment une énergie ultrasonore, thermique, radiofréquence, transportée par un faisceau de particules ionisantes, de neutrons, de rayons γ ou X.

Le procédé peut comporter l'étape selon laquelle une substance toxique ou non, des molécules toxiques ou non, et/ou des marqueurs spécifiques sont envoyés vers une région du corps d'où provient la luminescence détectée par la capsule afin de provoquer la destruction ou un ciblage des cellules ou de la zone tissulaire en lien avec l'origine de la luminescence.

Cette étape d'envoi d'une substance, de molécules et/ou de marqueurs peut suivre ou non une étape de transmission de données de la capsule à un récepteur extérieur au corps humain ou animal dans lequel la détection a lieu.

Un procédé de traitement du corps humain ou animal est aussi décrit, dans lequel :
- une capsule autonome de détection est introduite dans le corps, cette capsule comportant au moins un détecteur optique et au moins un moyen permettant de transformer une énergie transmise par induction en chaleur,
- on reçoit de la capsule une information signalant la détection d'une luminescence,
- on soumet la capsule à un champ permettant d'élever sa température, dans le but de détruire les cellules à l'origine de la luminescence.

Le procédé peut être appliqué à l'animal et suivi du sacrifice de l'animal.

Un procédé pour vérifier l'efficacité d'un traitement thérapeutique est aussi décrit, dans lequel :
- on introduit dans le corps une capsule de détection autonome comportant un détecteur optique,
- on administre un marqueur, et
- on détecte une réponse du corps à ce marqueur à l'aide de la capsule.

Le procédé peut être appliqué à un animal et être suivi du sacrifice de l'animal.

Un procédé d'observation du pancréas est aussi décrit, dans lequel un détecteur optique est introduit dans le conduit digestif, ce détecteur optique étant sensible à une lumière de chimiluminescence ou de bioluminescence émise par le pancréas et observée à travers une paroi du conduit digestif par le détecteur optique.

Le procédé peut être appliqué à l'animal et suivi du sacrifice de l'animal.

Un procédé de détection de l'apparition de cellules cancéreuses est aussi décrit, dans lequel une capsule autonome de détection est introduite dans le corps, la capsule comportant un détecteur sensible à un phénomène lié à l'apparition de cellules cancéreuses,
procédé dans lequel le détecteur est périodiquement activé pour détecter la présence audit phénomène.

Ledit phénomène peut être une luminescence provoquée par l'emploi d'un marqueur. La luminescence peut être exogène ou autogène.

Le détecteur peut comporter un biocapteur.

Le détecteur peut comporter un capteur à amplificateur optique.

Le détecteur peut comporter une micro caméra.

L'analyse des données provenant du détecteur peut s'effectuer hors du corps.

Bien entendu, l'invention n'est pas limitée aux exemples qui viennent d'être décrits.

L'expression « comportant un » doit se comprendre comme étant synonyme de « comportant au moins un ».

## Revendications

1. Capsule (1) autonome de détection destinée à être introduite dans le corps humain ou animal, la capsule étant dépourvue de source lumineuse pour illuminer le champ d'observation, et comportant :
- un détecteur optique (4) agencé pour détecter une lumière émise par bioluminescence ou chimiluminescence, sans excitation lumineuse du champ d'observation du détecteur optique (4) par la capsule et,
- des moyens (3, 5) d'enregistrement, de traitement et/ou de transmission des données relatives à la lumière détectée par le détecteur optique (4).

2. Capsule selon la revendication 1, le détecteur optique (4) étant agencé pour détecter les radiations du pioche infrarouge.

3. Capsule selon l'une quelconque des revendications précédente, le détecteur optique (4) étant agencé pour observer dans un champ d'environ 180° autour d'un axe de la capsule.

4. Capsule selon la revendication 1 ou 2, le détecteur optique comportant une pluralité de capteurs recouvrant toute la surface de la capsule.

5. Capsule selon l'une quelconque des revendications 1 à 4, le détecteur optique comportant au moins un capteur à amplification optique.

6. Capsule selon l'une quelconque des revendications précédentes, la capsule comportant un émetteur radiofréquence (3) pour communiquer avec un récepteur externe situé à l'extérieur du corps humain ou animal dans lequel la détection a lieu et/ou la capsule permettant la libération d'une substance contenue dans un compartiment de la capsule.

7. Capsule selon l'une quelconque des revendications 1 à 6 le détecteur optique comportant une pluralité de capteurs optiques agencés pour localiser l'émission de lumière relativement à la capsule.

8. Capsule selon l'une quelconque des revendications précédentes, comportant un moyen de production de chaleur pour élever une région au moins de la surface extérieure de la capsule à une température supérieure ou égale à 40 °C.

9. Capsule selon la revendication 8, le moyen de production de chaleur comportant un anneau de court-circuit.

10. Capsule selon l'une quelconque des revendications 1 à 9, comportant un actuateur.

11. Capsule selon la revendication 10, l'actuateur augmentant une dimension externe de la capsule lorsqu'actionné.

12. Capsule selon l'une quelconque des revendications précédentes, présentant une forme aplatie.

13. Capsule selon l'une quelconque des revendications précédentes, comportant une mémoire électronique dans laquelle des données de détection sont enregistrées, un émetteur permettant de transférer à distance, sur requête externe, les données et un moyen pour déclencher automatiquement des phases de détection périodiquement.

14. Ensemble de capsules autonomes, comportant :
- une première capsule selon l'une quelconque des revendications 1 à 14 et,
- une deuxième capsule distincte de la première et agencée pour libérer une substance destinée à provoquer l'émission par chimiluminescence ou bioluminescence de lumière en présence d'une cible prédéfinie, cette lumière étant détectable par la première capsule.

15. Procédé de détection de luminescence au sein d'un animal autre que l'être humain, dans lequel on observe une bioluminescence ou chimiluminescence, sans excitation optique du champ d'observation, à l'aide d'un détecteur d'une capsule autonome de détection comportant une enveloppe permettant son introduction dans le corps humain ou animal, un détecteur optique à structure amplificatrice de la lumière, et des moyens d'enregistrement, de traitement et/ou de transmission de données relatives à la lumière détectée par le détecteur optique, l'observation étant précédée ou suivie d'un sacrifice de l'animal.

## Claims

1. A self-contained detection capsule (1) for insertion into the human or animal body, the capsule being free of a light source for illuminating the field of view, and comprising:
- an optical detector (4) arranged to detect a light emitted by bioluminescence or chemiluminescence, without light excitation of the field of view of the optical detector (4) by the capsule, and
- means (3, 5) for recording, processing and/or transmitting data relating to the light detected by the optical detector (4).

2. The capsule as claimed in claim 1, the optical detector (4) being arranged to detect near infrared radiation.

3. The capsule as claimed in either one of the preceding claims, the optical detector (4) being arranged to view a field of approximately 180° about an axis of the capsule.

4. The capsule as claimed in claim 1 or 2, the optical detector comprising a plurality of sensors covering the entire surface of the capsule.

5. The capsule as claimed in any one of claims 1 to 4, the optical detector comprising at least one sensor with optical amplification.

6. The capsule as claimed in any one of the preceding claims, comprising a radiofrequency emitter (3) for communicating with an external receiver located outside the human or animal body in which the detection is carried out and/or the capsule allowing the release of a substance contained in a compartment of the capsule.

7. The capsule as claimed in any one of claims 1 to 6, the optical detector comprising a plurality of optical sensors arranged to localize the emission of light relative to the capsule.

8. The capsule as claimed in any one of the preceding claims, comprising a heat-producing means for raising at least one region of the external surface of the capsule to a temperature of greater than or equal to 40°C.

9. The capsule as claimed in claim 8, the heat-producing means comprising a short-circuit ring.

10. The capsule as claimed in any one of claims 1 to 9, comprising an actuator.

11. The capsule as claimed in claim 10, the actuator increasing an external dimension of the capsule when actuated.

12. The capsule as claimed in any one of the preceding claims, which has a flattened shape.

13. The capsule as claimed in any one of the preceding claims, comprising an electronic memory in which detection data are recorded, an emitter for transferring the data remotely, upon external request, and means for automatically triggering detection phases periodically.

14. A set of self-contained capsules, comprising:
- a first capsule as claimed in any one of claims 1 to 14, and
- a second capsule, distinct from the first and arranged to release a substance intended to bring about the emission by chemiluminescence or bioluminescence of light in the presence of a predefined target, it being possible for this light to be detected by the first capsule.

15. A method for detecting luminescence inside an animal other than the human being, in which one observes a bioluminescence or a chemiluminescence, with optical excitation of the field of view, via a detector of a self-contained detection capsule comprising an envelop configured for introduction in the human or animal body, an optical detector with a light amplifying structure and means for recording, processing and/or transmitting data relative to light detected by the optical detector, the observation being preceded or followed by a sacrifice of the animal.

## Patentansprüche

1. Autonome Nachweiskapsel (1), die dazu bestimmt ist, in den menschlichen oder tierischen Körper eingeführt zu werden, wobei die Kapsel nicht über eine Lichtquelle zur Beleuchtung des Beobachtungsfeldes verfügt und aufweist:
- einen optischen Detektor (4), der dazu ausgebildet ist, Licht zu detektieren, das durch Biolumineszenz oder Chemolumineszenz emittiert wird, ohne Lichtanregung des Beobachtungsfeldes des optischen Detektors (4) durch die Kapsel, und
- Mittel (3, 5) zur Aufzeichnung, Verarbeitung und/oder Übermittlung von Daten bezüglich des von dem optischen Detektor (4) detektierten Lichts.

2. Kapsel nach Anspruch 1, bei der der optische Detektor (4) dazu ausgebildet ist, Strahlung im nahen Infrarot zu detektieren.

3. Kapsel nach einem der vorstehenden Ansprüche, bei der der Detektor (4) dazu ausgebildet ist ein Feld von etwa 180° um eine Achse der Kapsel herum zu observieren.

4. Kapsel anspruch 1 oder 2, bei der der optische Detektor mehrere Messaufnehmer aufweist, die die gesamte Oberfläche der Kapsel bedecken.

5. Kapsel nach einem der Ansprüche 1 bis 4, bei der der optische Detektor wenigstens einen Messaufnehmer mit optischer Verstärkung aufweist.

6. Kapsel nach einem der vorstehenden Ansprüche, bei der die Kapsel einen Sender (3) für Radiofrequenz zur Kommunikation mit einem externen Empfänger aufweist, der sich außerhalb des menschlichen oder tierischen Körpers befindet, in welchem die Detektion stattfindet, und/oder die Kapsel die Freisetzung einer Substanz erlaubt, die in einem Abteil der Kapsel enthalten ist.

7. Kapsel nach einem der Ansprüche 1 bis 6, bei der der optische Detektor mehrere optische Messaufnehmer aufweist, die dazu ausgebildet sind, die Emission von Licht relativ zu der Kapsel zu lokalisieren.

8. Kapsel nach einem der vorstehenden Ansprüche, mit einem Mittel zur Erzeugung von Wärme zum Erhöhen der Temperatur zumindest einer Region auf der äußeren Oberfläche der Kapsel auf einen Wert größer oder gleich 40°C.

9. Kapsel nach anspruch 8, bei der das Mittel zur Erzeugung von Wärme einen Kurzschlussring aufweist.

10. Kapsel nach einem der Ansprüche 1 bis 9, mit einem Aktuator.

11. Kapsel nach Anspruch 10, bei der der Aktuator bei Betätigung eine äußere Abmessung der Kapsel vergrößert.

12. Kapsel nach einem der vorstehenden Ansprüche, die eine abgeflachte Form hat.

13. Kapsel nach einem der vorstehenden Ansprüche, mit einem elektronischen Speicher, in dem Messdaten gespeichert werden, einem Sender, der es auf eine externe Anforderung hin erlaubt, die Daten zu übertragen, und einem Mittel zur automatischen periodischen Auslösung von Detektionsphasen.

14. Satz von autonomen Kapseln, mit:
- einer ersten Kapsel nach einem der Ansprüche 1 bis 14 und
- einer zweiten Kapsel, die von der ersten verschieden ist und dazu ausgebildet ist, eine Substanz freizusetzen, die dazu bestimmt ist, in Gegenwart eines vordefinierten Ziels die Emission von Licht durch Chemolumineszenz oder Biolumineszenz zu provozieren, wobei dieses Licht von der ersten Kapsel detektierbar ist.

15. Verfahren zur Detektion von Lumineszenz in einem Tier, mit Ausnahme von Menschen, bei dem man ohne optische Anregung des Beobachtungsfeldes eine Biolumineszenz oder Chemolumineszenz beobachtet, mit Hilfe eines Detektors einer autonomen Nachweiskapsel, die eine Hülle, die es erlaubt, sie in den menschlichen oder tierischen Körper einzuführen, einen optischen Detektor mit einer Struktur zur Lichtverstärkung, und Mittel zur Aufzeichnung, Verarbeitung und/oder Übermittlung von Daten aufweist, die sich auf das von dem optischen Detektor detektierte Licht beziehen, wobei die Beobachtung einer Tötung des Tieres vorangeht oder nachfolgt.
